(19) **Europäisches Patentamt / European Patent Office / Office européen des brevets**

(11) **EP 4 772 182 A1**

(12) # EUROPEAN PATENT APPLICATION
published in accordance with Art. 153(4) EPC

(43) Date of publication:
08.07.2026 Bulletin 2026/28

(21) Application number: 25789180.4

(22) Date of filing: 23.01.2025

(51) International Patent Classification (IPC):
*A61K 31/706* (2006.01)   *A23L 33/13* (2016.01)
*A61P 39/06* (2006.01)   *A61P 39/00* (2006.01)
*A61P 25/28* (2006.01)   *A61P 25/20* (2006.01)
*A61P 9/00* (2006.01)

(86) International application number:
PCT/CN2025/074350

(87) International publication number:
WO 2025/218307 (23.10.2025 Gazette 2025/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 17.04.2024 CN 202410466701

(71) Applicant: EffePharm (Shanghai) Co., Ltd.
Shanghai 201601 (CN)

(72) Inventors:
• SHEN, Qiang
Shanghai 201601 (CN)

• YU, Jianjun
Shanghai 201601 (CN)
• XU, Qinyuan
Shanghai 201601 (CN)
• JING, Eryong
Shanghai 201601 (CN)
• LI, Jiayan
Shanghai 201601 (CN)

(74) Representative: Barker Brettell LLP
100 Hagley Road
Edgbaston
Birmingham, West Midlands B16 8QQ (GB)

(54) **PHARMACEUTICAL USE OF REDUCED ß-NICOTINAMIDE MONONUCLEOTIDE**

(57)   Provided is pharmaceutical use of reduced β-nicotinamide mononucleotide (NMNH). Specifically, the present invention relates to use of reduced β-nicotinamide mononucleotide (NMNH) and/or a corresponding salt thereof in the preparation of a drug or health-care product for relieving fatigue, improving sleep, promoting heart health, and improving cognition and/or combating aging.

Figure 1

**Description**

**CROSS-REFERENCE TO RELATED APPLICATION**

The present application claims priority to Chinese Patent Application with the application No. 202410466701.8, entitled "PHARMACEUTICAL USE OF REDUCED β-NICOTINAMIDE MONONUCLEOTIDE", and filed to the China National Intellectual Property Administration on April 17, 2024, the entire contents of which are incorporated herein by reference.

**TECHNICAL FIELD**

The present application belongs to the technical field of pharmaceutical preparations, specifically relating to pharmaceutical use of a reduced β-nicotinamide mononucleotide, and more specifically relating to use of a reduced β-nicotinamide mononucleotide (NMNH) in the manufacture of drugs for relieving fatigue, improving sleep, promoting heart health, improving cognition and/or combating aging.

**BACKGROUND**

Aging is a gradual and irreversible pathophysiological process, primarily characterized by a decline in tissue and cellular function and a significantly increased risk of various age-related diseases, including neurodegenerative diseases, cardiovascular diseases, metabolic diseases, musculoskeletal diseases, and immune system diseases. While advancements in modern medicine have promoted human health and significantly extended life expectancy, with the aging of society, various chronic diseases are gradually becoming the leading cause of disability and death among the elderly. Currently, research on aging mainly focuses on elucidating how various endogenous and exogenous stressors participate in the regulation of aging, such as genomic instability, telomere dysfunction, epigenetic alterations, loss of protein homeostasis, impaired autophagy, mitochondrial dysfunction, cellular senescence, stem cell depletion, altered intercellular communication, and dysregulated nutrient sensing. Furthermore, there is an expectation in the art to conduct in-depth research into the pathogenesis of aging to identify interventions that promote health and longevity (such as calorie restriction, microbial transplantation, and nutritional intervention) and clinical treatments for age-related diseases (such as senescent cell depletion, stem cell therapy, and antioxidant and anti-inflammatory therapies).

Currently, nicotinamide adenine dinucleotide (NAD) is undoubtedly one of the most popular molecules in the field of combating aging, and has become central to the history of combating aging substances. NAD exists in two forms: the oxidized form (NAD+) and the reduced form (NADH). The oxidized form, NAD+, accepts a hydrogen ion to become the reduced form, NADH, a conversion process that is crucial for central carbon metabolism. As a coenzyme in redox reactions, NAD+ is an important component of energy metabolism; it is also an essential cofactor for non-redox NAD+-dependent enzymes, including sirtuins, CD38, and poly(adp-ribose) polymerases. NAD+ can directly or indirectly affect many key cellular functions, including metabolic pathways, DNA repair, chromatin remodeling, cellular senescence, and immune cell function. These cellular processes and functions are essential for maintaining tissue and metabolic homeostasis and healthy aging. Notably, in various model organisms, including rodents and humans, aging is typically accompanied by a gradual decline in tissue and cellular NAD+ levels. The decline in NAD+ levels is causally linked to many age-related diseases, including cognitive decline, cancer, metabolic diseases, sarcopenia, and frailty. Many age-related diseases can be slowed or even reversed by restoring NAD+ levels. Therefore, targeting NAD+ metabolism has become a potential treatment for improving age-related diseases and extending healthy lifespan.

NAD+ can be synthesized using tryptophan in the de novo biosynthesis pathway, using nicotinic acid (NA) in the preiss-handler pathway, and using nicotinamide (NAM), nicotinamide riboside (NR), nicotinamide mononucleotide (NMN), reduced nicotinamide ribose (NRH), or reduced nicotinamide mononucleotide (NMNH) in the salvage pathway. In particular, NAM, NR, NRH, and NMN, as key intermediates for NAD+, have been extensively studied for their potential therapeutic effects in many mouse disease models. NMN, in particular, is considered the most suitable NAD+ precursor and is currently enjoying strong market demand and consumer appeal globally. Therefore, the development of more novel drugs based on NAD+ activity for ameliorative and therapeutic effects is of positive significance.

**SUMMARY OF THE INVENTION**

Therefore, the technical problem to be solved by the present application is to provide a new pharmaceutical use of reduced β-nicotinamide mononucleotide (NMNH), and more specifically, it relates to use of reduced β-nicotinamide mononucleotide (NMNH) in the manufacture of drugs for relieving fatigue, improving sleep, promoting heart health, improving cognition and/or combating aging.

The second technical problem to be solved by the present application is to provide a drug that has the effects of relieving fatigue, improving sleep, promoting heart health, improving cognition and/or combating aging.

To address the aforementioned technical problems, the present application provides use of a reduced nicotinamide mononucleotide (NMNH) and/or a salt thereof in the manufacture of a preparation having at least one of the following effects (1)-(5):

(1) relieving fatigue;
(2) improving sleep;
(3) promoting heart health;
(4) improving cognition; and
(5) combating aging.

Specifically, the daily dose of the reduced nicotinamide mononucleotide (NMNH) and/or a salt thereof is 1 to 1000 mg per kg body weight.

Specifically, the salt of the reduced nicotinamide mononucleotide (NMNH) comprises any one or a mixture of two or more selected from the group consisting of sodium salt, calcium salt, magnesium salt, potassium salt, iron salt, zinc salt, and manganese salt.

Specifically, the preparation comprises food, drug and/or supplements.

Specifically, the preparation comprises at least one of an oral formulation, a dosage form of injection administration, a dosage form of respiratory tract administration, a dosage form of transdermal administration, a dosage form of mucosal administration, or a dosage form of cavity administration.

Specifically, the preparation comprises one or more of the following forms: tablets, capsules, granules, aqueous solutions, enteric-coated preparations, injections, slurries, emulsions, creams, foams, sprays, ointments, gels, lotions, pads, roll-on preparations, caplets, lozenges, sugar lozenges, chewable tablets, jellies, colloidal preparations, syrups, liquid solutions, suspensions, buccal films, sublingual films, oral adhesive films, powders, solid crystals, orally disintegrating tablets, or pastes.

Specifically, the preparation further comprises excipients or carriers acceptable for food, drug and/or supplements,

The present application also discloses a composition in which the active ingredient comprises reduced nicotinamide mononucleotide (NMNH) and/or a salt thereof.

The present application also discloses a food, drug and/or supplements having effects of relieving fatigue, improving sleep, promoting heart health, improving cognition, and/or combating aging, wherein the food, drug and/or supplements comprises reduced nicotinamide mononucleotide (NMNH) and/or salt thereof, or the composition.

Specifically, the food, drug and/or supplements having effects of relieving fatigue, improving sleep, promoting heart health, improving cognition, and/or combating aging has a content of reduced nicotinamide mononucleotide (NMNH) and/or a salt thereof ranging from 0.01 wt% to 100 wt%.

The present application also discloses a method for relieving fatigue, improving sleep, promoting heart health, improving cognition, and/or combating aging, comprising administering reduced nicotinamide mononucleotide (NMNH) and/or a salt thereof to a subject in need thereof.

Specifically, the daily dose of the reduced nicotinamide mononucleotide (NMNH) and/or a salt thereof is 1 to 1000 mg per kg body weight.

Specifically, the salt of the reduced nicotinamide mononucleotide (NMNH) comprises any one or a mixture of two or more selected from the group consisting of sodium salt, calcium salt, magnesium salt, potassium salt, iron salt, zinc salt, and manganese salt.

The present application utilizes reduced nicotinamide mononucleotide (NMNH) or a corresponding salt thereof as an active ingredient in the manufacture of drugs or supplements having effects of relieving fatigue, improving sleep, promoting heart health, improving cognition, and/or combating aging, effectively solving the problems of drugs or supplementss, such as poor efficacy, high cost, and toxic side effects.

By utilizing reduced nicotinamide mononucleotide (NMNH) or corresponding salt thereof as the active ingredient, the present application not only demonstrates its effects in relieving fatigue, improving sleep, promoting heart health, improving cognition, and combating aging, but also shows that compared with the same dose of NMN, reduced nicotinamide mononucleotide (NMNH) can more effectively increase NAD+ levels in the blood, brain, liver, kidneys, heart, and gastrocnemius muscle. This makes NMNH a more promising NAD+ enhancer with potential applications in the field of combating aging, effectively expanding the health application areas of NMNH.

**BRIEF DESCRIPTION OF THE DRAWINGS**

To make the content of the present application easier to understand, the following detailed description is provided based on specific embodiments and accompanying drawings.

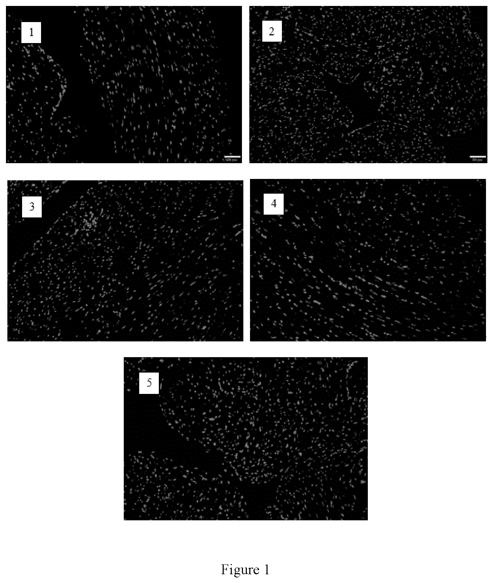

Figure 1 shows the staining results of cardiomyocytes under a fluorescence microscope, wherein 1 to 5 represent cardiomyocyte apoptosis in the blank control group, the positive control group-coenzyme Q10, the NMNH-100 mg/kg group, the NMNH-200 mg/kg group, and the NMNH-300 mg/kg group, respectively. Blue represents negatively stained cells, i.e., viable cardiomyocytes, and green represents positively stained cells, i.e., apoptotic cardiomyocytes.

## DETAILED DESCRIPTION

In the following examples of the present application, the test substance used in the studies on the properties of reduced β-nicotinamide mononucleotide (NMNH) is NMNH, prepared with pure water; the blank control is ultrapure water; all are prepared on the day of use and cannot be stored overnight.

In the following examples described in the present application, experimental mice were administered the test substance via oral gavage or intraperitoneal injection. Oral gavage is a method of administration in which the drug solution or suspension is directly instilled into the animal's stomach using a gavage needle. It is one of the most commonly used administration methods in animal experiments, particularly for animal modeling in physiological, pharmacological, and immunological studies, as well as for research on drug toxicology, efficacy, and dosage determination. Either a solution or a suspension is acceptable for gavage, as long as the drug remains homogeneous and stable, and it is suitable for repeated administration. The selection of gavage administration tools generally involves choosing different models of gavage needles depending on the age of the mice; for example, size 6 needles can be used for nude mice or young mice under 5 weeks old, size 8 or 9 needles can be used for mice weighing less than 30g, and size 12 needles can be used for mice weighing more than 30g.

In the following examples of the present application, the specific steps of the gavage experiment are as follows:

1. Experimental preparation: Fasting for 4-8 hours is required before gavage to ensure that excessive stomach contents do not hinder the gavage injection or affect the drug absorption rate.

2. Drug preparation: The drug is prepared according to the concentration required for each group, and the dosage is calculated based on the body weight of each group of mice.

3. Preparation for gavage: The mice are weighed, and the gavage needle and syringe are set up. The required dosage is drawn up according to the weight, and the prepared drug is set aside for later use.

4. Mouse handling and immobilization: The mouse is handled by grasping its tail, with stimulation minimized. Care is taken to avoid grasping the tail too close to the tip. The mouse is placed on a rough surface. The left hand (assuming the operator is right-handed) is used to grasp the skin behind the ears and neck, while the left palm and little/ring fingers are used to secure the tail, ensuring that the head, neck, and body (mouth and esophagus) are aligned in a straight line with the abdomen facing upward. The mouse must be firmly immobilized without pulling the neck skin too tightly. If the grasp is improper, the mouse is released and re-grasped immediately to avoid complications in subsequent procedures.

5. Injection and administration: The syringe is held in the right hand, and the needle is inserted into the oral cavity from one corner of the mouth, avoiding the teeth. The needle is gently inserted along the palate and into the lower esophagus, with care taken not to damage the esophagus. If resistance is encountered, the needle is gently slid up and down. When the mouse swallows, the cardiac sphincter muscles relax, and the resistance is felt to disappear, leaving a loss of resistance. The needle is then slowly advanced. Generally, when the needle has been inserted 3-4 cm into the mouse (approximately 3/4 of the gavage needle length) and no air backflow is observed when the syringe is pulled back, the needle is considered to have entered the stomach, and the injection may be administered. If the animal struggles violently, shows signs of respiratory distress, or if significant resistance is felt, the injection is stopped immediately and the needle is withdrawn. Another attempt may be made once the animal has calmed down. After the injection, the mouse is released and observed. If no abnormal breathing is noted, the gavage is considered successful.

In the following examples of the present application, dissecting tools, including forceps, scalpels, and scissors, are disinfected with 75% alcohol wipes and thoroughly rinsed with double-distilled water (ddH$_2$O); the area is disinfected by spraying with 70% ethanol; and PBS for cleaning tissue is prepared in a sterile culture dish.

Example 1: Experiment on Improving Sleep Function

The experiments in this example are based on "Guideline on Functional Testing and Evaluation of Health Food (2022 version)" and "Guideline on Testing and Evaluation of Health Food Functions (2022 version)".

Experimental principle: Sodium pentobarbital inhibits the transmission of excitatory impulses to the cerebral cortex, thereby exerting an inhibitory effect on the central nervous system. In experimental animals, low doses produce a hypnotic effect. The experiment aimed to observe whether the test drug could prolong sleep time, building upon the hypnotic effect of sodium pentobarbital.

The instruments and reagents involved in this example comprise mouse cages and sodium pentobarbital.

Experimental protocol

The experimental protocol for this example is shown in Table 1 below.

Table 1. Experimental Protocol for Improving Sleep

| Experimental Animals | Protocol | Group | Dosage/ kg·bw | Number of Animals | Days of Feeding |
|---|---|---|---|---|---|
| C57 pure line (inbred strain) mice, male (single sex), body weight 18-22g | Gavage: Prolon-gation of Pento-barbital Sodium-Indu ced Sleep Time Test | Blank group (ultra-pure water) | 20ml | 10 | 20 |
| | | Positive control group (melatonin) | 40mg | 10 | 20 |
| | | Low-concentration test substance group | 100mg | 10 | 20 |
| | | Medium-concen-tration test sub-stance group | 200mg | 9 | 20 |
| | | High-concentration test substance group | 300mg | 10 | 20 |

Experimental procedures

1. Preliminary Experiment

Before conducting animal experiments, a preliminary experiment was conducted to determine the sodium pentobarbital dosage that would induce 100% sleep in mice without causing them to sleep for an excessively long period.

2. Formal Experiment

C57 mice were administered oral gavage for 20 consecutive days. After the last administration, each group of mice was intraperitoneally injected with sodium pentobarbital at a dose of 50 mg/kg. After placement of the animals, the sleep duration of each mouse was recorded based on the loss of the righting reflex. The loss of the righting reflex was defined as the mouse remaining on its back for 30 seconds or more without exhibiting the righting reflex within 15 seconds.

In this example, the experimental results of each group are statistically summarized in Table 2 below.

Table 2. Statistical Results of Each Experimental Group

| Group | No. | Weight /g | Injection Volume/ μL | Administration Time | Sleep Onset Time | Sleep Duration /min | Sleep Duration/ min | Mean Sleep Duration/ min |
|---|---|---|---|---|---|---|---|---|
| Blank control group | 4 | 24.5 | 123 | 16:10 | 16:27 | 0:17 | 17 | 17.60 |
| | 3 | 23.9 | 119 | 16:11 | 16:29 | 0:18 | 18 | |
| | 2 | 26.21 | 131 | 16:13 | 16:30 | 0:17 | 17 | |
| | 1 | 26.4 | 132 | 16:14 | 16:33 | 0:19 | 19 | |
| | 5 | 24.6 | 123 | 16:15:00 | 16:35:0 0 | 0:20 | 20 | |
| | 6 | 25.7 | 129 | 16:16:00 | 16:32:0 0 | 0:16 | 16 | |
| | 7 | 28.2 | 141 | 16:16:00 | 16:34:0 0 | 0:18 | 18 | |
| | 8 | 25.6 | 128 | 16:17:00 | 16:33:0 0 | 0:16 | 16 | |
| | 9 | 24.30 | 122.00 | 0.68 | 0.69 | 0.01 | 17.00 | |
| | 10 | 24.60 | 123.00 | 0.68 | 0.69 | 0.01 | 18.00 | |
| Positive control group | 10 | 23.2 | 116 | 16:19 | 16:49 | 0:30 | 30 | 31.00 |
| | 5 | 24 | 120 | 16:20 | 16:50 | 0:30 | 30 | |
| | 4 | 24.6 | 123 | 16:21 | 16:50 | 0:29 | 30 | |
| | 8 | 26.6 | 13 | 16:22 | 16:53 | 0:31 | 31 | |
| | 1 | 25.1 | 125 | 16:22 | 16:55 | 0:33 | 33 | |
| | 6 | 25.06 | 125 | 16:24 | 16:59 | 0:35 | 35 | |
| | 3 | 24.2 | 121 | 16:27 | 16:55 | 0:28 | 28 | |
| | 9 | 27.6 | 138 | 16:28 | 16:56 | 0:28 | 28 | |
| | 2 | 25.8 | 129 | 16:29 | 17:03 | 0:34 | 34 | |
| | 7 | 25.6 | 128 | 16:30 | 17:01 | 0:31 | 31 | |
| NMNH 100mg/ kg | 3 | 24.8 | 124 | 16:36 | 17:12 | 0:36 | 36 | 36.60 |
| | 4 | 24.6 | 123 | 16:37 | 17:16 | 0:39 | 39 | |
| | 6 | 25.9 | 129 | 16:38 | 17:16 | 0:38 | 38 | |
| | 2 | 18.3 | 92 | 16:41 | 17:18 | 0:37 | 37 | |
| | 1 | 25.06 | 125 | 16:42 | 17:17 | 0:35 | 34 | |
| | 7 | 25.4 | 127 | 16:43 | 17:20 | 0:37 | 37 | |
| | 8 | 20.6 | 103 | 16:44 | 17:21 | 0:37 | 37 | |
| | 5 | 21 | 105 | 16:45 | 17:20 | 0:35 | 35 | |
| | 10 | 22.10 | 110.50 | 0.70 | 0.72 | 0.03 | 37.00 | |
| | 9 | 23.50 | 117.50 | 0.70 | 0.72 | 0.03 | 36.00 | |

(continued)

| Group | No. | Weight /g | Injection Volume/ μL | Administration Time | Sleep Onset Time | Sleep Duration /min | Sleep Duration/ min | Mean Sleep Duration/ min |
|---|---|---|---|---|---|---|---|---|
| NMNH 200mg/ kg | 4 | 21.22 | 106 | 15:53 | 16:33 | 0:40 | 40 | 38.33 |
| | 5 | 18.9 | 95 | 15:54 | 16:32 | 0:38 | 38 | |
| | 3 | 26 | 130 | 15:57 | 16:37 | 0:40 | 40 | |
| | 1 | 20.8 | 100 | 16:01 | 16:39 | 0:38 | 38 | |
| | 2 | 26.4 | 132 | 16:02 | 16:38 | 0:36 | 36 | |
| | 6 | 22 | 110 | 16:03 | 16:41 | 0:38 | 38 | |
| | 9 | 22.00 | 0.00 | 0.67 | 0.69 | 0.03 | 38.00 | |
| | 8 | 23.10 | 0.00 | 0.67 | 0.70 | 0.03 | 39.00 | |
| | 7 | 21.90 | 0.00 | 0.67 | 0.70 | 0.03 | 38.00 | |
| NMNH 300mg/ kg | 9 | 26.6 | 133 | 16:48 | 17:26 | 0:38 | 38 | 39.90 |
| | 4 | 25.1 | 125 | 16:49 | 17:28 | 0:39 | 39 | |
| | 5 | 28.1 | 140 | 16:50 | 17:30 | 0:40 | 40 | |
| | 7 | 19.7 | 98 | 16:50 | 17:29 | 0:39 | 39 | |
| | 1 | 26.2 | 131 | 16:51 | 17:32 | 0:41 | 41 | |
| | 3 | 16.6 | 83 | 16:52 | 17:33 | 0:41 | 41 | |
| | 6 | 25.7 | 128 | 16:53 | 17:33 | 0:40 | 40 | |
| | 2 | 26.3 | 131 | 16:54 | 17:33 | 0:39 | 39 | |
| | 8 | 27.2 | 136 | 16:54 | 17:36 | 0:42 | 42 | |
| | 10 | 26.30 | 131.50 | 0.70 | 0.73 | 0.03 | 40.00 | |

In this example, the P value results of each experimental group are statistically summarized in Table 3 below.

Table 3. Statistical P Value Results

| Dunnett's Comparison Test | Difference | 95.00% Confidence Interval | Below Critical Level | P Value |
|---|---|---|---|---|
| Blank group vs. Positive group | -13.4 | -15.17 to -11.63 | Yes | <0.0001 |
| Blank group vs. Low-concentration group | -19 | -20.77 to -17.23 | Yes | <0.0001 |
| Blank group vs. Medium-concentration group | -20.73 | -22.56 to -18.91 | Yes | <0.0001 |
| Blank group vs. High-concentration group | -22.3 | -24.07 to -20.53 | Yes | <0.0001 |

It is evident that, under experimental conditions, compared with the blank control group, both the positive control substance melatonin and different concentrations of reduced nicotinamide mononucleotide (NMNH) effectively improved sleep in mice, and the effect of the test substance NMNH in improving sleep showed a certain dose-dependent effect.

Example 2: Experiment on Relieving Fatigue

The testing in this example is based on "Guideline on Functional Testing and Evaluation of Health Food (2022 version)" and the "Guideline on Testing and Evaluation of Health Food Functions (2022 version)". Physical fatigue is a decrease in muscle strength caused by a series of biochemical changes in the body triggered by exercise. Therefore, there are two main methods for evaluating fatigue: endurance testing and the detection of biochemical changes.

The experimental protocol for this example is shown in Table 4 below.

Table 4. Experimental Protocol for Relieving Fatigue

| Experimental Animals | Protocol | Group | Dosage/ kg·bw | Number of Animals | Days of Feeding |
|---|---|---|---|---|---|
| C57 pure line (inbred strain) mice, half male and half female, 6 weeks old | Intraperitoneal injection: Weight-loaded swimming test; Blood lactate test | Blank group (ultrapure water) | 4μL | 9 | 15 |
| | | Positive control group (taurine) | 4μL *75mg/ml | 9 | 15 |
| | | NMNH-100mg/kg | 4μL *25mg/ml | 9 | 15 |
| | | NMNH-200mg/kg | 4μL *50mg/ml | 9 | 15 |
| | | NMNH-100mg/kg | 4μL *75mg/ml | 9 | 15 |

Weight-loaded swimming experiment

Experimental principle: The most prominent manifestation of fatigue is a decline in exercise endurance, which is the most direct and objective indicator of physical fatigue. The duration of swimming can reflect the degree of exercise fatigue in animals.

Experimental procedure: C57 mice were administered the test substance via intraperitoneal injection every other day for 15 days. 30 minutes after the last injection, the mice were placed in a swimming tank with a lead weight of 5% of their body weight attached to the base of their tails. The mice were then placed in the swimming tank with a water temperature of $25\pm1°C$ and a water depth of 40cm. The time from the start of swimming to death was recorded with a stopwatch, which is the weight-loaded swimming time of the mice.

In this example, the results of the weight-loaded swimming experiment are shown in Table 5 below.

Table 5. Results of the Weight-Loaded Swimming Experiment

| No. | Group | Swimming Time (minutes) |
|---|---|---|
| 1 | Blank control group | 62 |
| 2 | Positive control - taurine group | 76 |
| 3 | NMNH-100mg/kg | 78 |
| 4 | NMNH-200mg/kg | 89 |
| 5 | NMNH-300mg/kg | 93 |

Blood lactate measurement

The testing procedures were as follows:

1) Addition of standard: Standard wells and sample wells were set up. 50 μL of standard of different concentrations were added to each standard well;

2) Sample addition: Blank wells (which received no sample or enzyme-labeled reagent, with all other procedures being identical) and test sample wells were set up. In the test sample wells of the enzyme-labeled coated plate, 40 μL of sample diluent was added first, followed by 10 μL of the test sample (resulting in a final 5-fold dilution of the sample). Samples were added to the bottom of the wells, avoiding contact with the well walls as much as possible, and gently mixed by shaking;

3) Enzyme addition: Enzyme-labeled reagent (100 μL) was added to each well, except for the blank wells;

4) Incubation: The plate was sealed with sealing film and incubated at 37°C for 60 minutes;

5) Solution preparation: The 20-fold concentrated washing solution was diluted 20-fold with distilled water and set aside;

6) Washing: The sealing film was carefully removed, the liquid was discarded, and the plate was shaken dry. Each well was filled with washing solution, allowed to stand for 30 seconds, and then discarded. This process was repeated 5 times, followed by patting dry;

7) Color development: 50 μL of color developer A was added to each well, followed by 50 μl of color developer B. The plate was gently shaken to mix, and incubated at 37°C in the dark for 15 minutes.

8) Termination: 50 μl of stop solution was added to each well to terminate the reaction (at which point the blue color immediately turned yellow).

9) Measurement: The absorbance (OD value) of each well was measured sequentially at a wavelength of 450 nm, with blank wells used for zero adjustment. The measurement was performed within 15 minutes after adding the stop solution.

In this example, the blood lactate test data 15 days after administration are shown in Table 6 below.

Table 6. Results of Blood Lactate Measurement 15 Days After Administration

| Group | No. | A Value - A Blank | A Standard - A Blank | Standard Concentration mmol/L | Dilution Factor | Lactate Content mmol/L | Mean |
|---|---|---|---|---|---|---|---|
| Blank control group | 1 | 0.2402 | | | | 18.7943 | 18.557 |
| | 2 | 0.2351 | | | | 18.3953 | |
| | 3 | 0.2362 | | | | 18.4814 | |
| | 4 | 0.2055 | | | | 16.0796 | 16.1553 |
| | 5 | 0.2129 | | | | 16.6586 | |
| | 6 | 0.201 | | | | 15.7276 | |
| | 7 | 0.1992 | | | | 15.5868 | 19.4428 |
| | 8 | 0.1921 | | | | 15.0313 | |
| | 9 | 0.3542 | | | | 27.7104 | |
| Positive control group | 1 | 0.1697 | 0.1917 | 3 | 5 | 13.2789 | 13.2945 |
| | 2 | 0.1674 | | | | 13.0989 | |
| | 3 | 0.1726 | | | | 13.5057 | |
| | 4 | 0.1565 | | | | 12.2462 | 11.9697 |
| | 5 | 0.151 | | | | 11.8159 | |
| | 6 | 0.1514 | | | | 11.8472 | |
| | 7 | 0.1443 | | | | 11.2917 | 11.1196 |
| | 8 | 0.1409 | | | | 11.0257 | |
| | 9 | 0.1411 | | | | 11.0414 | |
| NMNH-100 mg/kg | 1 | 0.1418 | | | | 11.0961 | 11.0336 |
| | 2 | 0.1397 | | | | 10.9318 | |
| | 3 | 0.1415 | | | | 11.0727 | |
| | 4 | 0.1499 | | | | 11.7298 | 12.0605 |
| | 5 | 0.1531 | | | | 11.9802 | |
| | 6 | 0.1594 | | | | 12.4715 | |
| | 7 | 0.1214 | | | | 9.5002 | 9.8913 |
| | 8 | 0.1235 | | | | 9.6645 | |
| | 9 | 0.1343 | | | | 10.5094 | |
| NMNH-200 mg/kg | 1 | 0.1326 | | | | 10.3764 | 10.5042 |
| | 2 | 0.1389 | | | | 10.8693 | |

(continued)

| Group | No. | A Value - A Blank | A Standard - A Blank | Standard Concentration mmol/L | Dilution Factor | Lactate Content mmol/L | Mean |
|---|---|---|---|---|---|---|---|
| | 3 | 0.1312 | | | | 10.2669 | |
| | 4 | 0.1181 | | | | 9.242 | |
| | 5 | 0.122 | | | | 9.5471 | 9.4011 |
| | 6 | 0.1203 | | | | 9.4141 | |
| | 7 | 0.0716 | | | | 5.6041 | |
| | 8 | 0.0779 | | | | 6.097 | 5.8362 |
| | 9 | 0.0742 | | | | 5.8075 | |
| NMNH-300 mg/kg | 1 | 0.0678 | | | | 5.3069 | |
| | 2 | 0.0792 | | | | 6.1987 | 5.6198 |
| | 3 | 0.0684 | | | | 5.3538 | |
| | 4 | 0.1162 | | | | 9.0934 | |
| | 5 | 0.1084 | | | | 8.4831 | 8.9864 |
| | 6 | 0.1199 | | | | 9.3828 | |
| | 7 | 0.1183 | | | | 9.2576 | |
| | 8 | 0.1189 | | | | 9.3046 | 9.3489 |
| | 9 | 0.1212 | | | | 9.4845 | |

In this example, the P value results of the blood lactate test data 15 days after administration are shown in Table 7 below.

Table 7. Blood Lactate Data with P Values

| | Dunnett's Comparison Test | Difference | 95.00% Confidence Interval | Below Critical Level | P Value |
|---|---|---|---|---|---|
| 15 days after administration | Blank group vs. Positive group | 5.927 | 1.782 to 10.07 | Yes | 0.0068 |
| | Blank group vs. Low-concentration group | 7.06 | 2.915 to 11.21 | Yes | 0.0021 |
| | Blank group vs. Medium-concentratio n group | 9.473 | 5.328 to 13.62 | Yes | 0.0002 |
| | Blank group vs. High-concentration group | 10.07 | 5.922 to 14.21 | Yes | 0.0001 |

It is evident that, under experimental conditions, compared with the control group, taurine in the positive control group and different concentrations of reduced nicotinamide mononucleotide (NMNH) can effectively relieve fatigue in mice, prolong the weight-loaded swimming time, and reduce blood lactate levels. Moreover, the effect of relieving fatigue of the test substance NMNH is dose-dependent.

Example 3: Experiment on Promoting Heart Health

The testing methods used in this example comprise: "Guideline on Functional Testing and Evaluation of Health Food (2022 version)" and "Guideline on Testing and Evaluation of Health Food Functions (2022 version)".
Experimental Principle: Apoptosis detection is an excellent method for assessing cellular health and function, in which chromosomal DNA fragmentation is a gradual, phased process. Chromosomal DNA is first degraded into large fragments of 50-300 kb by endogenous nucleases. Then, approximately 30% of the chromosomal DNA is randomly cleaved between nucleosome units by $Ca^{2+}$ and $Mg^{2+}$-dependent endonucleases, forming 180-200 bp nucleosome DNA polymers. Therefore, in the late stages of apoptosis, DNA is degraded into 180-200 bp fragments, exposing numerous 3'-OH ends on the fragmented genomic DNA. Terminal deoxynucleotidyl transferase (TdT) is a template-independent DNA polymerase that catalyzes the binding of deoxyribonucleotides to the 3'-OH ends of the fragmented DNA molecules. Therefore, the TUNEL (TdT-mediated dUTP Nick End Labeling) apoptosis test can detect the fragmentation of nuclear DNA in tissue cells during the late stages of apoptosis. The principle is that, under the action of TdT enzyme, fluorescently labeled dUTP (FITC-12-dUTP) is incorporated into the 3'-OH end exposed when genomic DNA breaks, so that it can be detected by fluorescence microscopy or flow cytometry (FITC excitation at 450-500 nm, emission at 515-565 nm).

Experimental protocol

The experimental protocol for this example is shown in Table 8 below.

Table 8. Experimental Protocol

| Experimental Animals | Protocol | Group | Dosage/ kg·bw | Number of Animals | Days of Feeding |
|---|---|---|---|---|---|
| C57 pure line (inbred strain) mice, half male and half female, body weight 18-22g | Cardiomyocyte apoptosis experiment (TUNEL fluorescence) | Blank group (ultrapure water) | 20ml | 8 | 20 |
| | | Positive control group (Coenzyme Q10) | 40mg | 8 | 20 |
| | | Low-concentration test substance group | 100mg | 8 | 20 |
| | | Medium-concentration test substance group | 200mg | 8 | 20 |
| | | High-concentration test substance group | 300mg | 8 | 20 |

The experimental procedures were as follows:

1. C57 mice were orally administered different concentrations of the test sample, positive control, or ultrapure water by gavage for 20 consecutive days. They were then euthanized with saturated carbon dioxide, and heart tissue was collected and subjected to the following treatment: dewaxed in xylene for 5-10 minutes, then transferred to fresh xylene and dewaxed again for 5-10 minutes; followed by rehydration in anhydrous ethanol for 5 minutes, 90% ethanol for 2 minutes, 70% ethanol for 2 minutes, and distilled water for 2 minutes.
2. The sections were incubated with 20 μg/mL DNase-free Proteinase K (Note: Beyotime's ST532/ST533 Proteinase K (20 mg/mL) is recommended. A 20 μg/mL DNase-free Proteinase K working solution can be prepared by diluting the stock 1000-fold with P0106 Immunostaining Wash Buffer or 10 mM Tris-HCl, pH 7.4-7.8). The incubation was carried out at 20-37°C for 15-30 minutes (the optimal incubation temperature and time for different tissues need to be optimized by the user).
3. The sections were washed three times with PBS or HBSS. Note: It is crucial to thoroughly remove the Proteinase K in this step, as residual enzyme can significantly interfere with subsequent labeling reactions.
4. Preparation of TUNEL test solution: An appropriate amount of TUNEL test solution was prepared as shown below, and mixed thoroughly. Note: The prepared TUNEL test solution must be used immediately and cannot be frozen for storage.

For 1 sample: 5 μL of TdT enzyme, 45 μL of fluorescent labeling solution, 50 μL of TUNEL test working solution total.
For 5 samples: 25 μL of TdT enzyme, 225 μL of fluorescent labeling solution, 250 μL of TUNEL test working solution total.
For 10 samples: 50 μL of TdT enzyme, 450 μL of fluorescent labeling solution, 500 μL of TUNEL test working solution total.
5. 50 μL of the TUNEL test working solution was added to each sample. The samples were then incubated at 37°C for 60 minutes, protected from light.
6. The samples were washed three times with PBS or HBSS.
7. The samples were mounted with an anti-fade mounting medium and observed under a fluorescence microscope. The excitation wavelength range is 450-500 nm, and the emission wavelength range is 515-565 nm (green fluorescence).

In this example, the staining results of cardiomyocytes under a fluorescence microscope are shown in Figure 1. Apoptotic cardiomyocytes are stained green, and surviving cardiomyocytes are stained blue. The apoptosis status of cardiomyocytes is further assessed by calculating the proportion of green cells.

In this example, the statistical results are shown in Table 9 below.

Table 9. Experimental Statistical Results

| Group | No. | Total Cells | TUNEL-Positive Cells | Apoptosis Rate (%) | Mean Apoptosis Rate (%) | Group |
|---|---|---|---|---|---|---|
| Blank control group | 1-1 | 902 | 62 | 6.87% | 8.99% | Blank group |
| | 1-2 | 754 | 126 | 16.71% | | |
| | 2-1 | 1018 | 98 | 9.63% | | |
| | 2-2 | 905 | 99 | 10.94% | | |
| | 3-1 | 1134 | 114 | 10.05% | | |
| | 3-2 | 545 | 62 | 11.38% | | |
| | 4-1 | 1179 | 57 | 4.83% | | |
| | 4-2 | 663 | 91 | 13.73% | | |
| | 5-1 | 900 | 50 | 5.56% | | |
| | 5-2 | 1241 | 47 | 3.79% | | |
| | 6-1 | 1399 | 79 | 5.65% | | |
| | 6-2 | 1280 | 47 | 3.67% | | |
| | 7-1 | 897 | 15 | 1.67% | | |
| | 7-2 | 690 | 33 | 4.78% | | |
| | 8-1 | 1357 | 323 | 23.80% | | |
| | 8-2 | 1113 | 120 | 10.78% | | |
| Positive control group | 1-1 | 838 | 8 | 0.95% | 4.23% | Positive group |
| | 1-2 | 1036 | 7 | 0.68% | | |
| | 2-1 | 1234 | 67 | 5.43% | | |
| | 2-2 | 1570 | 238 | 15.16% | | |
| | 3-1 | 1157 | 34 | 2.94% | | |
| | 3-2 | 1110 | 70 | 6.31% | | |
| | 4-1 | 1944 | 13 | 0.67% | | |
| | 4-2 | 1644 | 6 | 0.36% | | |
| | 5-1 | 1177 | 6 | 0.51% | | |
| | 5-2 | 1009 | 7 | 0.69% | | |
| | 6-1 | 1597 | 24 | 1.50% | | |
| | 6-2 | 766 | 40 | 5.22% | | |
| | 7-1 | 1079 | 183 | 16.96% | | |
| | 7-2 | 1175 | 99 | 8.43% | | |
| | 8-1 | 1455 | 19 | 1.31% | | |
| | 8-2 | 1108 | 6 | 0.54% | | |

(continued)

| Group | No. | Total Cells | TUNEL-Positive Cells | Apoptosis Rate (%) | Mean Apoptosis Rate (%) | Group |
|---|---|---|---|---|---|---|
| NMN-H-100m-g/k g | 1-1 | 1476 | 8 | 0.54% | 0.43% | Low-concentration |
| | 1-2 | 1022 | 3 | 0.29% | | |
| | 2-1 | 1293 | 3 | 0.23% | | |
| | 2-2 | 899 | 13 | 1.45% | | |
| | 3-1 | 1099 | 15 | 1.36% | | |
| | 3-2 | 1188 | 1 | 0.08% | | |
| | 4-1 | 1114 | 9 | 0.81% | | |
| | 4-2 | 868 | 3 | 0.35% | | |
| | 5-1 | 1245 | 1 | 0.08% | | |
| | 5-2 | 1171 | 2 | 0.17% | | |
| | 6-1 | 1267 | 2 | 0.16% | | |
| | 6-2 | 1112 | 8 | 0.72% | | |
| | 7-1 | 966 | 3 | 0.31% | | |
| | 7-2 | 896 | 1 | 0.11% | | |
| | 8-1 | 1102 | 3 | 0.27% | | |
| | 8-2 | 1125 | 0 | 0.00% | | |
| NMNH 200mg/k g | 1-1 | 1121 | 8 | 0.71% | 0.27% | Medium-concentration |
| | 1-2 | 1041 | 4 | 0.38% | | |
| | 2-1 | 873 | 0 | 0.00% | | |
| | 2-2 | 1136 | 2 | 0.18% | | |
| | 3-1 | 885 | 8 | 0.90% | | |
| | 3-1 | 1146 | 5 | 0.44% | | |
| | 4-1 | 745 | 1 | 0.13% | | |
| | 4-1 | 1030 | 0 | 0.00% | | |
| | 5-1 | 1258 | 2 | 0.16% | | |
| | 5-2 | 1142 | 0 | 0.00% | | |
| | 6-1 | 970 | 3 | 0.31% | | |
| | 6-2 | 748 | 2 | 0.27% | | |
| | 7-1 | 1208 | 3 | 0.25% | | |
| | 7-2 | 1098 | 5 | 0.46% | | |
| | 8-1 | 1300 | 0 | 0.00% | | |
| | 8-2 | 1192 | 2 | 0.17% | | |

(continued)

| Group | No. | Total Cells | TUNEL-Positive Cells | Apoptosis Rate (%) | Mean Apoptosis Rate (%) | Group |
|---|---|---|---|---|---|---|
| NMNH 300mg/kg | 1-1 | 1469 | 0 | 0.00% | 0.11% | High-concentration |
| | 1-2 | 1390 | 0 | 0.00% | | |
| | 2-1 | 1162 | 1 | 0.09% | | |
| | 2-2 | 855 | 1 | 0.12% | | |
| | 3-1 | 913 | 2 | 0.22% | | |
| | 3-2 | 1448 | 0 | 0.00% | | |
| | 4-1 | 1135 | 5 | 0.44% | | |
| | 4-2 | 1155 | 0 | 0.00% | | |
| | 5-1 | 1248 | 2 | 0.16% | | |
| | 5-2 | 1180 | 2 | 0.17% | | |
| | 6-1 | 1023 | 1 | 0.10% | | |
| | 6-2 | 1272 | 0 | 0.00% | | |
| | 7-1 | 1278 | 1 | 0.08% | | |
| | 7-2 | 1005 | 2 | 0.20% | | |
| | 8-1 | 1486 | 1 | 0.07% | | |
| | 8-2 | 1282 | 1 | 0.08% | | |

In this example, the P value results of each experimental group are shown in Table 10 below.

Table 10. P Value Results

| Dunnett's Comparison Test | Difference | 95.00% Confidence Interval | Below Critical Level | P Value |
|---|---|---|---|---|
| Blank group vs. Positive group | 0.04938 | 0.01915 to 0.07960 | Yes | 0.0005 |
| Blank group vs. Low-concentration group | 0.08875 | 0.05852 to 0.1190 | Yes | <0.0001 |
| Blank group vs. Medium-concentration group | 0.09063 | 0.06040 to 0.1209 | Yes | <0.0001 |
| Blank group vs. High-concentration group | 0.09188 | 0.06165 to 0.1221 | Yes | <0.0001 |

It is evident that, under experimental conditions, compared with the blank control group, both the positive control substance coenzyme Q10 and different concentrations of reduced nicotinamide mononucleotide (NMNH) can effectively reduce the apoptosis rate of cardiomyocytes and have a cardioprotective effect. Moreover, the cardioprotective effect of NMNH is better than that of coenzyme Q10 and is dose-dependent.

Example 4: Experiment on Improving Cognition

In this example, the testing standards comprise: "Guideline on Functional Testing and Evaluation of Health Food (2022 version)" and "Guideline on Testing and Evaluation of Health Food Functions (2022 version)".

Acetylcholine, an important neurotransmitter in the brain, is the biochemical basis of memory and learning. Studies have confirmed that acetylcholinesterase (AChE) can break down acetylcholine at synapses, thereby affecting individual learning and memory activities. Patients with cognitive impairment have elevated serum AChE levels. Normally, superoxide dismutase (SOD) and malondialdehyde (MDA) are in balance. However, in individuals with cognitive impairment, MDA is abnormally elevated. SOD is an important oxygen free radical scavenger that effectively scavenges oxygen free radicals, preventing cell damage. Abnormally elevated MDA levels, exceeding the SOD clearance range, can lead to increased oxidative stress, increased release of oxygen free radicals, damage to brain cells and neurons, and resulting in cholinergic pathway disorders, cholinergic dysfunction, and decreased cholinergic neurotransmitter levels in cognitively related brain regions. Monitoring changes in acetylcholinesterase (AChE) and MDA levels can reflect their impact on cognition.

Malondialdehyde (MDA), a lipid peroxidation degradation product, can condense with thiobarbituric acid (TBA) to form a red product with a maximum absorption peak at 532 nm. Because the substrate is thiobarbituric acid (TBA), this method is called the TBA method. Acetylcholinesterase hydrolyzes acetylcholine to produce choline and acetic acid. Choline reacts with a thiol-based chromogenic agent to form a yellow compound, TNB (symmetric trinitrobenzene). Quantification is performed colorimetrically based on the color intensity; the amount of choline in the hydrolysis product reflects the activity of cholinesterase.

The instruments used in this example comprise a microplate reader (Tecan; Sunrise); reagents and consumables comprise a malondialdehyde (MDA) detection kit, a 96-well microplate; an acetylcholinesterase (AChE) detection kit, and a 96-well microplate.

Experimental Protocol

The experimental protocol for this example is shown in Table 11 below.

Table 11. Experimental Protocol

| Experimental Animals | Protocol | Group | Dosage/ kg·bw | Number of Animals | Days of Feeding |
|---|---|---|---|---|---|
| C57 pure line (inbred strain) mice, half male and half female, body weight 18-22g | Serum AChE detection test; Serum MDA detection test | Blank group (ultrapure water) | 20ml | 10 | 20 |
| | | Positive control group (Pyrroloquinoline Quinone, PQQ) | 40mg | 10 | 20 |
| | | Low-concentration test substance group | 100mg | 9 | 20 |
| | | Medium-concentration test substance group | 200mg | 10 | 20 |
| | | High-concentration test substance group | 300mg | 10 | 20 |

The specific experimental procedures in this example comprised:

1. Sample pretreatment:

C57 mice were orally administered via gavage for 20 consecutive days. Serum/plasma was collected for direct measurement. If the measurement range was exceeded, the sample could be diluted with physiological saline before loading.

2. Experimental procedures:

Blank tubes, standard tubes, test tubes, and control tubes were set up according to the sample loading tables shown in Tables 12-13 below. Corresponding reagents were then added to the wells of a microplate according to the operating procedure. The centrifuge tubes were capped, a small hole was pierced in each cap with a needle, and the contents were mixed using a vortex mixer. The tubes were incubated in a 95°C water bath (or boiled in an uncovered pot) for 40 minutes. After incubation, the tubes were cooled under running water and then centrifuged at 3500-4000 rpm for 10 minutes (if centrifuging at speeds below 3000 rpm, centrifugation time should be extended to ensure complete precipitation). The supernatant was collected, and the absorbance of each tube was measured at 532 nm with a 1 cm light path, using distilled water for zero adjustment.

Table 12. Malondialdehyde (MDA) Sample Loading Table

| | Blank Tube | Standard Tube | Test Tube | Control Tube** |
|---|---|---|---|---|
| 10 nmol/mL Standard (mL) | | a* | | |
| Anhydrous ethanol (mL) | a* | | | |
| Test sample (mL) | | | a* | a* |
| Reagent 1 (mL) | a* | a* | a* | a* |

(continued)

| Mix well by shaking the rack several times | | | | |
|---|---|---|---|---|
| Reagent 2 (mL) | 3 | 3 | 3 | 3 |
| Reagent 3 (mL) | 1 | 1 | 1 | |
| 50% Glacial acetic acid (mL) | | | | 1 |
| [Note]: a*: it indicates that the amounts of sample, standard, anhydrous ethanol, and reagent 1 are all equal. (a* is generally 0.1-0.2 mL.) For example, if the sample is 0.1 mL, then the standard, anhydrous ethanol, and reagent 1 should also be 0.1 mL. If the sample is 0.2 mL, then the standard, anhydrous ethanol, and reagent 1 should also be 0.2 mL. Because absorbance is directly proportional to the amount of sample added, the results are not affected. **: generally, only 1-2 standard tubes, blank tubes, and control tubes are needed per batch. If the sample does not show hemolysis or lipemia, the control tube can be omitted and a blank tube can be used instead. ***: when aspirating the supernatant for colorimetric analysis, it is best to use a pipette to add the supernatant to the cuvette, avoiding pouring as much as possible to prevent precipitation from entering the cuvette and affecting the absorbance. | | | | |

Calculaion formula: MDA content in serum (plasma) = (measured OD value - control OD value) / (standard OD value - blank OD value) * standard concentration * dilution factor.

Table 13. Acetylcholinesterase (AChE) Sample Loading Table

| | Test Tube | Control Tube | Standard Tube | Blank Tube |
|---|---|---|---|---|
| Sample(mL) | a* | | | |
| 1 $\mu$mol/mL Standard Working Solution (mL) | | | a* | |
| Double-Distilled Water (mL) | | | | a* |
| Substrate Buffer (mL) | 0.5 | 0.5 | 0.5 | 0.5 |
| Chromogenic Working Solution (mL) | 0.5 | 0.5 | 0.5 | 0.5 |
| Mix well, react accurately at 37°C for 6 minutes | | | | |
| Inhibitor (mL) | 0.03 | 0.03 | 0.03 | 0.03 |
| Clearing Agent (mL) | 0.1 | 0.1 | 0.1 | 0.1 |
| Sample (mL) | | a* | | |
| Mix well, let stand for 15 minutes, measure OD value at 412 nm with 0.5 cm light path, zero with double-distilled water | | | | |
| [Note]: 1. Control tubes must be tested for each sample, as the absorbance of each control tube varies considerably. 2. Before colorimetric analysis, place the test tubes at room temperature for 15 minutes. If the room temperature is too low, precipitation or turbidity may occur. In this case, place the test tubes in a 37°C water bath for a short time to clarify the turbidity. Colorimetric analysis can only be performed after clarification, and this will not affect the experimental results. 3. Due to the short reaction time, the number of samples tested in each batch should not be too large. Accurately control the reaction time, otherwise it will affect the accuracy of the experiment. 4. The sample volume, standard volume, and double-distilled water volume indicated by a* are all equal: ① Serum (plasma) should be diluted 10 times with physiological saline before testing, with a reference sample volume of 30-50 $\mu$L; ② The reference sample volume for 10% brain tissue homogenate is 30-50 $\mu$L; ③ For whole blood diluted 1:99, take 0.1 mL of the solution and shake well before each sample. | | | | |

Calculation formula:

(1) Definition: Each milliliter of serum sample is kept at 37°C for 6 minutes, and 1 $\mu$mol of matrix in the hydrolysis reaction system is one activity unit.

(2) Calculation formula:

Serum (plasma) AChE = (A test - A control) / (A standard - A blank) * C * N activity (U/mL);

C standard: Standard concentration, 1 $\mu$mol/mL;

N: Dilution factor of the sample before testing.

In this example, the results of the cognitive improvement experiment are shown in Table 14 below.

Table 14. Results of Experiment on Improving Cognition

| Group | No. | MDA | | AChE | |
|---|---|---|---|---|---|
| | | Mean Content $\mu$mol/mL | Mean Content by Group $\mu$mol/mL | Mean Content $\mu$mol/mL | Mean Content by Group $\mu$mol/mL |
| Blank control group | 1 | 6.4068 | 6.9344 | 41.9745 | 44.6646 |
| | 2 | 6.1669 | | 39.3447 | |
| | 3 | 4.9916 | | 39.9829 | |
| | 4 | 10.4963 | | 41.3656 | |
| | 5 | 7.7129 | | 46.3354 | |
| | 6 | 6.1952 | | 45.1604 | |
| | 7 | 6.6916 | | 48.4077 | |
| | 8 | 6.8142 | | 54.7458 | |
| | 9 | 5.9721 | | 41.7879 | |
| | 10 | 7.8967 | | 47.5413 | |
| Positive control group | 1 | 5.9351 | 5.6490 | 40.7199 | 36.2653 |
| | 2 | 6.2976 | | 31.3877 | |
| | 3 | 5.4706 | | 31.5769 | |
| | 4 | 7.0350 | | 47.3330 | |
| | 5 | 4.9936 | | 32.1923 | |
| | 6 | 7.1505 | | 40.3147 | |
| | 7 | 3.8986 | | 32.5745 | |
| | 8 | 4.2484 | | 37.3058 | |
| | 9 | 5.7472 | | 34.3022 | |
| | 10 | 5.7131 | | 34.9461 | |
| NMNH 100mg/kg | 1 | 5.8217 | 5.2050 | 37.9698 | 35.3317 |
| | 2 | 5.2385 | | 36.3539 | |
| | 3 | 3.6104 | | 32.1136 | |
| | 4 | 3.2146 | | 44.7739 | |
| | 5 | 4.8569 | | 20.8592 | |
| | 6 | 5.0972 | | 27.9764 | |
| | 7 | 5.4718 | | 45.3473 | |
| | 8 | 5.6243 | | 24.9712 | |
| | 9 | 7.9093 | | 47.6197 | |

(continued)

| Group | No. | MDA | | AChE | |
|---|---|---|---|---|---|
| | | Mean Content $\mu$mol/mL | Mean Content by Group $\mu$mol/mL | Mean Content $\mu$mol/mL | Mean Content by Group $\mu$mol/mL |
| NMNH 200mg/kg | 1 | 4.1265 | 5.2007 | 37.3636 | 39.8644 |
| | 2 | 4.3986 | | 38.1642 | |
| | 3 | 9.9416 | | 38.8764 | |
| | 4 | 5.6090 | | 38.6384 | |
| | 5 | 3.2783 | | 39.4883 | |
| | 6 | 4.0258 | | 42.2612 | |
| | 7 | 4.8736 | | 44.5155 | |
| | 8 | 5.3521 | | 39.6077 | |
| | 9 | 5.3920 | | 41.0977 | |
| | 10 | 5.0094 | | 38.6311 | |
| NMNH 300mg/kg | 1 | 4.7729 | 5.2344 | 45.9753 | 41.6890 |
| | 2 | 2.8266 | | 43.1617 | |
| | 3 | 4.4618 | | 39.7838 | |
| | 4 | 3.9678 | | 39.7382 | |
| | 5 | 5.1566 | | 37.8215 | |
| | 6 | 7.9228 | | 38.9488 | |
| | 7 | 7.0077 | | 44.0065 | |
| | 8 | 5.7586 | | 44.0765 | |
| | 9 | 5.1222 | | 40.4557 | |
| | 10 | 5.3466 | | 42.9223 | |

In this example, the statistical P value of the experimental results is shown in Table 15 below.

Table 15. P Value results

| | Dunnett's Comparison Test | Difference | 95.00% Confidence Interval | Below Critical Level | P Value |
|---|---|---|---|---|---|
| Malondialdehyde (MDA) | Blank group vs. Positive group | 1.286 | -0.3689 to 2.941 | No | 0.1683 |
| | Blank group vs. Low-concentration group | 1.731 | 0.03030 to 3.431 | Yes | 0.045 |
| | Blank group vs. Medium-concentration group | 1.734 | 0.07910 to 3.389 | Yes | 0.0374 |
| | Blank group vs. High-concentration group | 1.7 | 0.04510 to 3.355 | Yes | 0.0424 |
| Acetylcholinesterase (AChE) | Blank group vs. Positive group | 8.4 | 2.281 to 14.52 | Yes | 0.0042 |
| | Blank group vs. Low-concentration group | 9.334 | 3.047 to 15.62 | Yes | 0.0019 |

(continued)

| | Dunnett's Comparison Test | Difference | 95.00% Confidence Interval | Below Critical Level | P Value |
|---|---|---|---|---|---|
| | Blank group vs. Medium-concentration group | 4.8 | -1.319 to 10.92 | No | 0.1623 |
| | Blank group vs. High-concentration group | 2.975 | -3.144 to 9.094 | No | 0.553 |

It is evident that, under experimental conditions, compared with the control group, both the positive control substance pyrroloquinoline quinone (PQQ) and low-dose reduced nicotinamide mononucleotide (NMNH) could effectively reduce acetylcholinesterase (AChE) levels, demonstrating the potential to improve cognition. Although the positive control group reduced malondialdehyde (MDA) levels, the difference was not statistically significant. Furthermore, medium and high doses of NMNH did not show a better ability to reduce MDA and AChE levels, which are positively correlated with cognitive impairment. The reasons for this are currently unclear and further research is needed to explore its molecular mechanism.

Example 5 Experiment on Combating Aging

The testing in this example is based on "Guideline on Functional Testing and Evaluation of Health Food (2022 version)" and "Guideline on Testing and Evaluation of Health Food Functions (2022 version)".
Experimental principle: Cell growth, senescence, and apoptosis are closely related to telomeres. Telomeres gradually shorten with continuous cell division. When they shorten to a certain extent, cell division is inhibited, replication ability is lost, and apoptosis occurs. Telomerase can catalyze the synthesis of telomere DNA, counteracting or delaying telomere shortening. The degree of cell senescence can be detected by measuring the length and rate of telomere loss. The fluorescence signal of telomere products amplified using telomere (T) primers is compared with that of single-copy gene products amplified using single-copy gene (S) primers. Two reaction tubes containing equal amounts of sample are used to simultaneously amplify T and S, and the fluorescence signals of the products are detected. The relative telomere length can be obtained using specific analysis software.
The instruments and reagents involved in this example comprise: a quantitative PCR instrument (ABI; Quantstudio7), an ultra-micro spectrophotometer (Thermo; Nanodrop One), a centrifuge (Eppendorf; 5425), and a vortex mixer (Beijing Dalong; MX-F); the reagents and consumables involved comprise a mouse telomere qPCR detection kit, a fluorescent quantitative PCR plate, and a sealing film (ABI).
The experimental protocol for this example is shown in Table 16 below.

Table 16. Experimental Protocol

| Experimental Animals | Protocol | Group | Dosage/ kg·bw | Number of Animals | Days of Feeding |
|---|---|---|---|---|---|
| C57 pure line (inbred strain) mice, half male and half female, body weight 18-22 g | Relative telomere length (T/S) | Blank group (ultra-pure water) | 20ml | 9 | 20 |
| | | Positive control group (lipoic acid) | 40mg | 8 | 20 |
| | | Low-concentration test substance group | 100mg | 8 | 20 |
| | | Medium-concentration test substance group | 200mg | 8 | 20 |
| | | High-concentration test substance group | 300mg | 8 | 20 |

The specific experimental procedures in this example comprised:

1. C57 mice were administered by oral gavage for 20 consecutive days, and blood samples were collected from the ophthalmic venous plexus for testing.

2. Blood DNA extraction and quality inspection: Blood DNA was extracted according to the instructions of the TianGen Kit (DP348). The extracted DNA was quality controlled using Nanodrop One. The purity of the DNA was considered acceptable when the A260/A280 ratio was approximately 1.8. The DNA content was calculated, and the DNA was dissolved in TE and stored in an ultra-low temperature freezer for subsequent use.

3. Quantitative Real-time PCR (qPCR) Detection: The reaction system is shown in Table 17 below. The reaction protocol comprised: 95°C for 3 min, 95°C for 30 sec, 50°C for 1 min, 72°C for 30 sec, for a total of 40 cycles.

Table 17. PCR Reaction System

| Component/Sample | Test Sample | Standard | Negative Control |
|---|---|---|---|
| 2*PCR MIX | 10 $\mu$L | 10 $\mu$L | 10 $\mu$L |
| Primer set | 5 $\mu$L | 5 $\mu$L | 5 $\mu$L |
| Standard | | 5 $\mu$L | |
| ddH$_2$O | | | 5 $\mu$L |
| Test Sample | 5 $\mu$L | | |
| Note: The Primer set can detect telomere genes and internal reference genes simultaneously in one reaction. The standard is a telomere repeat DNA template of a specific length. | | | |

It should be noted that the telomere length calculation method (T/S) is as follows: First, calculate $\Delta CT = CT$ (telomere) - CT (internal reference) for the standard and the sample respectively, and then calculate T/S = 2 - ^($\Delta CT$ (sample) - $\Delta CT$ (standard)).

Note: T represents the test sample, S represents the standard, and T/S is the ratio of the relative telomere lengths of the test sample to the standard.

In this example, the relative telomere length of the experimental samples and the statistical analysis results are shown in Table 18.

Table 18. Relative Telomere Length

| Group | No. | Target 2 | Target 1 | $\Delta CT$ | $\Delta CT$ (Sampl e) - $\Delta CT$ (Standa rd) | | T/S | Mean T/S |
|---|---|---|---|---|---|---|---|---|
| | 1 | 8.475 | 15.844 | -7.369 | -1.212 | 1.212 | 2.316 | |
| | 2 | 8.547 | 15.575 | -7.028 | -0.870 | 0.870 | 1.828 | |
| | 3 | 8.181 | 15.321 | -7.140 | -0.982 | 0.982 | 1.976 | |
| | 4 | 8.580 | 15.840 | -7.260 | -1.102 | 1.102 | 2.147 | |
| | 5 | 8.340 | 15.495 | -7.155 | -0.998 | 0.998 | 1.997 | 1.961 |
| | 6 | 8.401 | 15.359 | -6.958 | -0.800 | 0.800 | 1.742 | |
| | 7 | 7.970 | 15.256 | -7.286 | -1.128 | 1.128 | 2.186 | |
| Blank con-trol group | 8 | 8.605 | 15.519 | -6.913 | -0.755 | 0.755 | 1.688 | |
| | 9 | 8.487 | 15.466 | -6.979 | -0.821 | 0.821 | 1.767 | |

(continued)

| Group | No. | Target 2 | Target 1 | ΔCT | ΔCT (Sample) - ΔCT (Standard) | | T/S | Mean T/S |
|---|---|---|---|---|---|---|---|---|
| Positive control group | 1 | 8.095 | 15.534 | -7.440 | -1.282 | 1.282 | 2.431 | 2.265 |
| | 2 | 8.405 | 15.598 | -7.193 | -1.035 | 1.035 | 2.050 | |
| | 3 | 8.187 | 15.674 | -7.487 | -1.329 | 1.329 | 2.513 | |
| | 4 | 8.107 | 15.418 | -7.311 | -1.153 | 1.153 | 2.224 | |
| | 5 | 8.432 | 15.609 | -7.177 | -1.019 | 1.019 | 2.027 | |
| | 6 | 8.439 | 15.582 | -7.143 | -0.985 | 0.985 | 1.979 | |
| | 7 | 8.414 | 16.016 | -7.602 | -1.445 | 1.445 | 2.722 | |
| | 8 | 8.573 | 15.848 | -7.276 | -1.118 | 1.118 | 2.170 | |
| NMNH 100mg/kg | 1 | 8.404 | 15.914 | -7.510 | -1.352 | 1.352 | 2.553 | 2.307 |
| | 2 | 8.355 | 15.852 | -7.497 | -1.339 | 1.339 | 2.530 | |
| | 3 | 8.221 | 15.595 | -7.373 | -1.216 | 1.216 | 2.322 | |
| | 4 | 8.517 | 15.793 | -7.276 | -1.118 | 1.118 | 2.170 | |
| | 5 | 8.187 | 15.602 | -7.415 | -1.257 | 1.257 | 2.390 | |
| | 6 | 8.265 | 15.342 | -7.077 | -0.919 | 0.919 | 1.891 | |
| | 7 | 8.466 | 15.892 | -7.426 | -1.268 | 1.268 | 2.409 | |
| | 8 | 8.184 | 15.474 | -7.290 | -1.132 | 1.132 | 2.192 | |
| NMNH 200mg/kg | 1 | 8.284 | 15.764 | -7.480 | -1.322 | 1.322 | 2.500 | 2.394 |
| | 2 | 8.260 | 15.878 | -7.618 | -1.460 | 1.460 | 2.751 | |
| | 3 | 8.736 | 16.159 | -7.423 | -1.265 | 1.265 | 2.404 | |
| | 4 | 8.535 | 15.562 | -7.027 | -0.870 | 0.870 | 1.827 | |
| | 5 | 8.375 | 15.772 | -7.397 | -1.240 | 1.240 | 2.361 | |
| | 6 | 8.162 | 15.665 | -7.503 | -1.345 | 1.345 | 2.541 | |
| | 7 | 8.603 | 16.111 | -7.508 | -1.350 | 1.350 | 2.550 | |
| | 8 | 8.181 | 15.489 | -7.308 | -1.150 | 1.150 | 2.219 | |
| NMNH 300mg/kg | 1 | 8.665 | 15.999 | -7.334 | -1.176 | 1.176 | 2.259 | 2.368 |
| | 2 | 8.290 | 16.027 | -7.737 | -1.579 | 1.579 | 2.988 | |
| | 3 | 8.201 | 15.860 | -7.659 | -1.501 | 1.501 | 2.831 | |
| | 4 | 8.306 | 15.577 | -7.271 | -1.113 | 1.113 | 2.163 | |
| | 5 | 8.347 | 15.356 | -7.009 | -0.852 | 0.852 | 1.804 | |
| | 6 | 8.324 | 15.621 | -7.298 | -1.140 | 1.140 | 2.203 | |
| | 7 | 8.364 | 15.851 | -7.487 | -1.329 | 1.329 | 2.513 | |
| | 8 | 8.346 | 15.629 | -7.283 | -1.125 | 1.125 | 2.181 | |
| standard | Sample 64 | 9.328 | 15.486 | -6.158 | | | | |

In this example, the statistical results of the P value of each experimental group are shown in Table 19 below.

Table 19. Statistical Analysis Results

| Dunnett's Comparison Test | Difference | 95.00% Confidence Interval | Below Critical Level | P Value |
|---|---|---|---|---|
| Blank group vs. Control group | -0.3037 | -0.6515 to 0.04404 | No | 0.1020 |

(continued)

| Dunnett's Comparison Test | Difference | 95.00% Confidence Interval | Below Critical Level | P Value |
|---|---|---|---|---|
| Blank group vs. Low-concentration group | -0.3463 | -0.6941 to 0.001416 | No | 0.0512 |
| Blank group vs. Medium-concentration group | -0.4333 | -0.7811 to -0.08558 | Yes | 0.0106 |
| Blank group vs. High-concentration group | -0.4070 | -0.7547 to -0.05921 | Yes | 0.0174 |

It is evident that, under the experimental conditions, $\alpha$-lipoic acid, which possesses strong free radical scavenging capabilities, did not significantly prolong the relative length of telomeres after 20 days of oral administration. The same result occurred in the low-dose group of reduced nicotinamide mononucleotide (NMNH). The medium-dose and high-dose NMNH groups exhibited a significant ability to prolong the relative length of telomeres, suggesting a potential for delaying aging or alleviating age-related diseases.

Example 6

The present application relates to food, drug and/or supplements having effects of relieving fatigue, improving sleep, promoting heart health, improving cognition, and/or combating aging, using reduced nicotinamide mononucleotide (NMNH) and/or a salt thereof as active ingredients. The salt of the reduced nicotinamide mononucleotide (NMNH) comprise any one or a mixture of two or more selected from the group consisting of sodium salt, calcium salt, magnesium salt, potassium salt, iron salt, zinc salt, and manganese salt. Optionally, the content of the reduced nicotinamide mononucleotide (NMNH) and/or a salt thereof is in a range from 0.01 wt% to 100 wt%.

The present application relates to food, drug and/or supplements having effects of relieving fatigue, improving sleep, promoting heart health, improving cognition, and/or combating aging. These products can be formulated into conventional dosage forms known in the art, including an oral formulation, a dosage form of injection administration, a dosage form of respiratory tract administration, a dosage form of transdermal administration, a dosage form of mucosal administration, or a dosage form of cavity administration. Specific dosage forms comprise tablets, capsules, granules, aqueous solutions, enteric-coated preparations, injections, slurries, emulsions, creams, foams, sprays, ointments, gels, lotions, pads, roll-on preparations, caplets, lozenges, sugar lozenges, chewable tablets, jellies, colloidal preparations, syrups, liquid solutions, suspensions, buccal films, sublingual films, oral adhesive films, powders, solid crystals, orally disintegrating tablets, or pastes, or one or more of these forms.

In this example, the food, drug and/or supplements having effects of relieving fatigue, improving sleep, promoting heart health, improving cognition and/or combating aging is preferably an enteric-coated capsule or enteric-coated tablet, i.e. an enteric-coated preparation, while the excipients can be any raw materials known in the art.

Obviously, the above embodiments are merely illustrative examples for clear explanation and are not intended to limit the implementation. Those skilled in the art will recognize that other variations or modifications can be made based on the above description. It is neither necessary nor possible to exhaustively list all possible implementations here. However, obvious variations or modifications derived therefrom are still within the scope of protection of the present invention.

**Claims**

1.   Use of a reduced nicotinamide mononucleotide (NMNH) and/or a salt thereof in the manufacture of a preparation having at least one of the following effects (1)-(5):

    (1) relieving fatigue;
    (2) improving sleep;
    (3) promoting heart health;
    (4) improving cognition; and
    (5) combating aging.

2.   The use according to claim 1, wherein the daily dose of the reduced nicotinamide mononucleotide (NMNH) and/or a salt thereof is 1 to 1000 mg per kg body weight.

3.   The use according to claim 1 or 2, wherein the salt of the reduced nicotinamide mononucleotide (NMNH) comprises any one or a mixture of two or more selected from the group consisting of sodium salt, calcium salt, magnesium salt, potassium salt, iron salt, zinc salt, and manganese salt.

4.  The use according to any one of claims 1-3, wherein the preparation comprises food, drug and/or supplements.

5.  The use according to any one of claims 1-4, wherein the preparation comprises at least one of an oral formulation, a dosage form of injection administration, a dosage form of respiratory administration, a dosage form of transdermal administration, a dosage form of mucosal administration, or a dosage form of cavity preparation administration.

6.  The use according to claim 5, wherein the preparation comprises one or more of the following forms: tablets, capsules, granules, aqueous solutions, enteric-coated preparations, injections, slurries, emulsions, creams, foams, sprays, ointments, gels, lotions, pads, roll-on preparations, caplets, lozenges, sugar lozenges, chewable tablets, jellies, colloidal preparations, syrups, liquid solutions, suspensions, buccal films, sublingual films, oral adhesive films, powders, solid crystals, orally disintegrating tablets, or pastes.

7.  The use according to any one of claims 1-6, wherein the preparation further comprises excipients or carriers acceptable for food, drug and/or supplements.

8.  A composition, wherein the active ingredient of the composition comprises reduced nicotinamide mononucleotide (NMNH) and/or a salt thereof.

9.  A food, drug and/or supplements having effects of relieving fatigue, improving sleep, promoting heart health, improving cognition, and/or combating aging, wherein the food, drug and/or supplements comprises reduced nicotinamide mononucleotide (NMNH) and/or a salt thereof, or the composition of claim 8.

10. The food, drug and/or supplements having effects of relieving fatigue, improving sleep, promoting heart health, improving cognition, and/or combating aging according to claim 9, wherein the content of the reduced nicotinamide mononucleotide (NMNH) and/or a salt thereof is in a range from 0.01 wt% to 100 wt%.

11. A method for relieving fatigue, improving sleep, promoting heart health, improving cognition, and/or combating aging, comprising administering reduced nicotinamide mononucleotide (NMNH) and/or a salt thereof to a subject in need thereof.

12. The method according to claim 11, wherein the daily dose of the reduced nicotinamide mononucleotide (NMNH) and/or salt thereof is 1 to 1000 mg per kg body weight.

13. The method according to claim 11 or 12, wherein the salt of the reduced nicotinamide mononucleotide (NMNH) comprises any one or a mixture of two or more selected from the group consisting of sodium salt, calcium salt, magnesium salt, potassium salt, iron salt, zinc salt, and manganese salt.

Figure 1

## INTERNATIONAL SEARCH REPORT

| International application No. |
| --- |
| **PCT/CN2025/074350** |

| A. | CLASSIFICATION OF SUBJECT MATTER |
| --- | --- |

A61K 31/706(2006.01)i; A23L 33/13(2016.01)i; A61P 39/06(2006.01)i; A61P 39/00(2006.01)i; A61P 25/28(2006.01)i; A61P 25/20(2006.01)i; A61P 9/00(2006.01)i

According to International Patent Classification (IPC) or to both national classification and IPC

| B. | FIELDS SEARCHED |
| --- | --- |

Minimum documentation searched (classification system followed by classification symbols)

IPC: A61K,A23L,A61P

Documentation searched other than minimum documentation to the extent that such documents are included in the fields searched

Electronic data base consulted during the international search (name of data base and, where practicable, search terms used)

CNABS: WPABSC; DWPI; WPABS; VEN; CNTXT; EPTXT; USTXT; WOTXT; CNKI: 中国药物专利数据库, CHINESE PHARMACEUTICAL PATENT DATABASE; 百度学术搜索, Baidu Scholar Search; ISI-WEB OF SCIENCE; Pubmed: 还原型烟酰胺单核苷酸, 还原型, 烟酰胺单核苷酸, 疲劳, 睡眠, 心脏, 心肌, 认知, 衰老, Reduced Nicotinamide Mononucleotide, NMNH, NMN, fatigue, sleep, heart, myocardium, cognition, aging, ageing

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 118319935 A (EFFEPHARM (SHANGHAI) CO., LTD.) 12 July 2024 (2024-07-12) claims 1-10 | 1-13 |
| X | CN 113490676 A (TSINGHUA UNIVERSITY) 08 October 2021 (2021-10-08) claims 5 and 12, and description, paragraphs 6 and 135 | 8-10 |
| Y | CN 114432259 A (BRIGHT FUTURE PHARMACEUTICAL (SUZHOU) CO., LTD.) 06 May 2022 (2022-05-06) paragraphs 24 and 34 | 1-7, 11-13 |
| Y | CN 108025187 A (NEWSOUTH INNOVATIONS PTY LIMITED) 11 May 2018 (2018-05-11) claims 2 and 22-23 | 1-7, 11-13 |
| Y | CN 104814974 A (BONTAC BIO-ENGINEERING (SHENZHEN) CO., LTD.) 05 August 2015 (2015-08-05) claims 1-6, and description, paragraph 13 | 1-7, 11-13 |

| ✓ Further documents are listed in the continuation of Box C. | ✓ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **17 March 2025** | **24 March 2025** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2025/074350**

### C.    DOCUMENTS CONSIDERED TO BE RELEVANT

| Category* | Citation of document, with indication, where appropriate, of the relevant passages | Relevant to claim No. |
|---|---|---|
| Y | CN 117018009 A (INSTITUTE OF ENVIRONMENTAL MEDICINE AND OCCUPATIONAL MEDICINE, INSTITUTE OF MILITARY MEDICINE, ACADEMY OF MILITARY SCIENCES) 10 November 2023 (2023-11-10)<br>claims 1 and 9-10, and abstract | 1-7, 11-13 |
| Y | CN 113101298 A (SHENZHEN HOPELIFE BIOTECHNOLOGY CO., LTD.) 13 July 2021 (2021-07-13)<br>claims 1-8 | 1-7, 11-13 |
| Y | CN 113143946 A (TSINGHUA UNIVERSITY; THE AFFILIATED HOSPITAL OF QINGDAO UNIVERSITY; QINGDAO CANCER INSTITUTE) 23 July 2021 (2021-07-23)<br>claims 1-7, and description, paragraph 7 | 1-7, 11-13 |
| Y | CN 113332304 A (SHENZHEN HOPELIFE BIOTECHNOLOGY CO., LTD.) 03 September 2021 (2021-09-03)<br>claims 1-7 | 1-7, 11-13 |
| Y | CN 114344209 A (HEALTH AND HAPPINESS (H&H) HONG KONG LIMITED) 15 April 2022 (2022-04-15)<br>description, paragraphs 69-156 | 1-7, 11-13 |
| Y | CN 115820551 A (BIOTECH (SHENYANG) BIO-MEDICAL GROUP CO., LTD.) 21 March 2023 (2023-03-21)<br>claims 1-10, and description, paragraphs 3-4 | 1-7, 11-13 |
| Y | WO 2018052020 A1 (TANAKA MEGUMI; TANAKA TSUNEMARU) 22 March 2018 (2018-03-22)<br>claims 1-5, and description, paragraphs 22 and 41-42 | 1-7, 11-13 |
| Y | CN 113490676 A (TSINGHUA UNIVERSITY) 08 October 2021 (2021-10-08)<br>claims 5 and 12, and description, paragraphs 6 and 135 | 1-7, 11-13 |

Form PCT/ISA/210 (second sheet) (July 2022)

## INTERNATIONAL SEARCH REPORT

| International application No. |
|---|
| **PCT/CN2025/074350** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **11-13**
   because they relate to subject matter not required to be searched by this Authority, namely:

   The subject matter of claims 11-13 relates to a method for relieving fatigue, improving sleep, promoting heart health, improving cognition and/or resisting aging, and falls within treatment methods for a living human or animal body. According to PCT Rule 39.1(iv), no search can be carried out. The amended subject matter that is reasonably expected is pharmaceutical use claims corresponding to said claims. The present international search report is provided on the basis of the above amended subject matter that is reasonably expected.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2025/074350**

| Patent document cited in search report | | | Publication date (day/month/year) | Patent family member(s) | | | Publication date (day/month/year) |
|---|---|---|---|---|---|---|---|
| CN | 118319935 | A | 12 July 2024 | None | | | |
| CN | 113490676 | A | 08 October 2021 | WO | 2021098725 | A1 | 27 May 2021 |
| CN | 114432259 | A | 06 May 2022 | CN | 114432259 | B | 03 March 2023 |
| CN | 108025187 | A | 11 May 2018 | PL | 3288637 | T3 | 19 December 2022 |
| | | | | DK | 3288637 | T3 | 17 October 2022 |
| | | | | CA | 2984379 | A1 | 03 November 2016 |
| | | | | CA | 2984379 | C | 11 June 2024 |
| | | | | ES | 2927894 | T3 | 11 November 2022 |
| | | | | AU | 2016255853 | A1 | 30 November 2017 |
| | | | | AU | 2016255853 | B2 | 05 August 2021 |
| | | | | PT | 3288637 | T | 11 October 2022 |
| | | | | US | 2020405736 | A1 | 31 December 2020 |
| | | | | US | 11389469 | B2 | 19 July 2022 |
| | | | | US | 2018125871 | A1 | 10 May 2018 |
| | | | | US | 10603334 | B2 | 31 March 2020 |
| | | | | KR | 20180006389 | A | 17 January 2018 |
| | | | | JP | 2018514548 | A | 07 June 2018 |
| | | | | JP | 7193232 | B2 | 20 December 2022 |
| | | | | EP | 3288637 | A1 | 07 March 2018 |
| | | | | EP | 3288637 | A4 | 13 February 2019 |
| | | | | EP | 3288637 | B1 | 14 September 2022 |
| | | | | JP | 2021073207 | A | 13 May 2021 |
| | | | | WO | 2016176437 | A1 | 03 November 2016 |
| CN | 104814974 | A | 05 August 2015 | US | 2017266213 | A1 | 21 September 2017 |
| | | | | WO | 2016145911 | A1 | 22 September 2016 |
| CN | 117018009 | A | 10 November 2023 | None | | | |
| CN | 113101298 | A | 13 July 2021 | None | | | |
| CN | 113143946 | A | 23 July 2021 | None | | | |
| CN | 113332304 | A | 03 September 2021 | None | | | |
| CN | 114344209 | A | 15 April 2022 | CN | 114344209 | B | 20 October 2023 |
| CN | 115820551 | A | 21 March 2023 | None | | | |
| WO | 2018052020 | A1 | 22 March 2018 | EP | 3513796 | A1 | 24 July 2019 |
| | | | | EP | 3513796 | A4 | 06 May 2020 |
| | | | | US | 2020009170 | A1 | 09 January 2020 |
| | | | | JPWO | 2018052020 | A1 | 24 June 2019 |
| | | | | JP | 6803915 | B2 | 23 December 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410466701

**Non-patent literature cited in the description**

- *Guideline on Functional Testing and Evaluation of Health Food*, 2022

- *Guideline on Testing and Evaluation of Health Food Functions*, 2022